# EUROPÄISCHE PATENTSCHRIFT

(11) **EP 3 725 344 B1**
(45) Veröffentlichungstag und Bekanntmachung des Hinweises auf die Patenterteilung: **03.09.2025**
(21) Anmeldenummer: 20165570.1
(22) Anmeldetag: 25.03.2020
(51) Int. Cl.: A61M 5/14

(54) **TROPFKAMMERANORDNUNG FÜR EIN MEDIZINISCHES INFUSIONSSYSTEM UND VERFAHREN ZUR HERSTELLUNG EINER SOLCHEN TROPFKAMMERANORDNUNG**
DRIP CHAMBER ARRANGEMENT FOR A MEDICAL INFUSION SYSTEM AND METHOD FOR PRODUCING SUCH A DRIP CHAMBER ARRANGEMENT
AGENCEMENT DE CHAMBRE DE GOUTTEMENT POUR UN SYSTÈME DE PERFUSION MÉDICALE ET PROCÉDÉ DE PRODUCTION D'UN TEL AGENCEMENT DE CHAMBRE DE GOUTTEMENT

(30) Priorität: 18.04.2019 DE 102019205662
(43) Veröffentlichungstag der Anmeldung: 21.10.2020
(73) Patentinhaber: B. Braun Melsungen AG, 34212 Melsungen (DE)
(72) Erfinder: Hogrebe, Thilo, 22763 Hamburg (DE); Marquardsen, Jörg, 37235 Hessisch Lichtenau (DE); Schlitt, Christof, 34621 Obergrenzebach (DE); Seidel, Gerrit, 34121 Kassel (DE)
(74) Vertreter: Patentanwälte Ruff, Wilhelm, Beier, Dauster & Partner mbB

(56) Entgegenhaltungen:
- DE-A1- 19 931 092
- US-A- 4 198 971
- US-A1- 2018 177 942
- US-A1- 2018 304 009

## Beschreibung

Die Erfindung betrifft eine Tropfkammeranordnung für ein medizinisches Infusionssystem, aufweisend ein Tropfkammeroberteil, das einen Einlass zur Einleitung eines medizinischen Fluids in eine Tropfkammer aufweist, und ein Tropfkammerunterteil, das einen Auslass zur Ableitung des medizinischen Fluids aus der Tropfkammer aufweist, wobei das Tropfkammeroberteil und das Tropfkammerunterteil mittels einer fluiddichten ersten Fügeverbindung unter Ausbildung der Tropfkammer zusammengefügt sind. Die Erfindung betrifft zudem Verfahren zur Herstellung einer solchen Tropfkammeranordnung.

Eine derartige Tropfkammeranordnung ist im Bereich der Medizintechnik allgemein bekannt, siehe beispielsweise DE 199 31 092 A1 oder US 2018/304009 A1, und als Komponente eines Infusionssystems, das auch als Überleitungssystem bezeichnet werden kann, zur Verwendung bei einer Infusionstherapie vorgesehen. Die bekannte Tropfkammeranordnung ist mehrteilig ausgebildet und weist insoweit ein Tropfkammeroberteil und ein fluiddicht mit diesem zusammengefügtes Tropfkammerunterteil auf. Die beiden Teile der Tropfkammeranordnung umgrenzen eine Tropfkammer zur Aufnahme und Ableitung eines im Rahmen der Infusionstherapie zu verabreichenden medizinischen Fluids. Das Tropfkammeroberteil ist einer Einlassseite zugeordnet und weist dementsprechend einen Einlass zur Einleitung des medizinischen Fluids in die Tropfkammer auf. Das Tropfkammerunterteil ist demgegenüber einer Auslassseite zugeordnet und weist einen Auslass zur Ableitung des medizinischen Fluids aus der Tropfkammer auf. Die fluiddichte Fügeverbindung zwischen dem Tropfkammeroberteil und dem Tropfkammerunterteil ist bei der bekannten Tropfkammeranordnung mittels eines Umspritzungsprozesses ausgebildet, bei dem die beiden Teile an einem Außenumfang mit Kunststoff umspritzt werden. Ein bei diesem Umspritzungsprozess ausgebildeter Abschnitt kann eine Art Handhabungselement bilden, an dem die Tropfkammeranordnung gegriffen werden kann. Der Umspritzungsprozess ist fertigungstechnisch aufwändig und orientiert sich in erster Linie an den Erfordernissen der zu erreichenden fluiddichten Fügeverbindung.

Aufgabe der Erfindung ist es, eine Tropfkammeranordnung und ein Verfahren der eingangs genannten Art bereitzustellen, die eine verbesserte konstruktive Anpassbarkeit an ergonomische Erfordernisse bei der Handhabung und gleichzeitig eine kostengünstige Fertigung mit möglichst gleich getakteten Fertigungsschritten ermöglichen.

Diese Aufgabe wird für die Tropfkammeranordnung dadurch gelöst, dass ein separat von der ersten Fügeverbindung hergestelltes Handhabungselement vorgesehen ist, das die Tropfkammer in Umfangsrichtung wenigstens abschnittsweise ringförmig umgreift. Durch die erfindungsgemäße Lösung kann somit auf ein fertigungstechnisch aufwändiges und investitionsintensives Umspritzen von Tropfkammeroberteil und Tropfkammerunterteil zur gleichzeitigen Ausbildung der ersten Fügeverbindung und des Handhabungselements verzichtet werden. Zudem kann durch die erfindungsgemäße Lösung eine in verbesserter Weise an ergonomische Erfordernisse angepasste Anordnung und Gestaltgebung des Handhabungselements erreicht werden. Dies wird durch die separate Herstellung und Anordnung des Handhabungselements erreicht, wodurch nunmehr nicht mehr zwingend eine Anordnung im Bereich der ersten Fügeverbindung vorgesehen sein muss. Auch die Gestaltgebung des Handhabungselements kann aufgrund der erfindungsgemäßen Lösung unabhängig von den Erfordernissen der herzustellenden fluiddichten ersten Fügeverbindung erfolgen. Das Tropfkammeroberteil ist vorzugsweise formstabil und blickdurchlässig ausgebildet, wobei die blickdurchlässige Ausbildung einer leichten visuellen Kontrolle einer Tropfrate des medizinischen Fluids dient. Der Einlass des Tropfkammeroberteils ist zur fluidleitenden Verbindung mit einem Einstechteil vorgesehen. Das Tropfkammeroberteil kann unmittelbar an dem Einstechteil angeordnet und/oder einstückig mit diesem ausgebildet sein. Alternativ kann der Einlass mittels einer Schlauchleitung des Infusionssystems mit einem Auslass des Einstechteils fluidleitend verbindbar sein. Das Tropfkammerunterteil ist vorzugsweise formnachgiebig ausgebildet, wobei die formnachgiebige Ausbildung einer Pumpfunktion zum intialen Ansaugen des medizinischen Fluids durch den Einlass beim Ingangsetzen der Infusion dient. Die fluiddichte erste Fügeverbindung ist eine stoffschlüssige Fügeverbindung, die beispielsweise eine Schweiß- oder Klebeverbindung sein kann. Als zur Ausbildung der ersten Fügeverbindung besonders geeignete Verfahren haben sich Kaltverschweißen und Lösemittelkleben erwiesen. Diese Verfahren sind als solche grundsätzlich bekannt. Das Handhabungselement ist separat von der ersten Fügeverbindung hergestellt und demnach insbesondere nicht mittels eines Umspritzens der Tropfkammer mit Kunststoff ausgebildet. Stattdessen ist das Handhabungselement als gesonderte Komponente der Tropfkammeranordnung in einem gesonderten Fertigungsschritt gefertigt und ist hiernach mit den übrigen Komponenten der Tropfkammeranordnung zusammengefügt. Vorzugsweise ist das Handhabungselement aus Kunststoff gefertigt. Eine besonders ergonomische Handhabung kann erreicht werden, wenn das Handhabungselement als Weichkomponente aus einem formnachgiebigen Kunststoff gefertigt ist. Das Handhabungselement kann form-, kraft- und/oder stoffschlüssig mit einem Außenwandabschnitt des Tropfkammeroberteils und/oder mit einem Außenwandabschnitt des Tropfkammerunterteils zusammengefügt sein. Eine weiter verbesserte Ergonomie wird erreicht, wenn eine Außenkontur des Handhabungsteils die übrigen Komponenten der Tropfkammeranordnung in Radialrichtung der Tropfkammer nach außen überragt. Mit anderen Worten ausgedrückt bildet das Handhabungselement die dickste Stelle der Tropfkammeranordnung und kann auf diese Weise besonders einfach und sicher und mithin besonders ergonomisch manuell gegriffen werden.

In Ausgestaltung der Erfindung ist das Handhabungselement mittels einer formschlüssigen zweiten Fügeverbindung mit einem Außenwandabschnitt des Tropfkammeroberteils und/oder mit einem Außenwandabschnitt des Tropfkammerunterteils zusammengefügt. Die formschlüssige zweite Fügeverbindung kann formschlüssig in Umfangs-, Radial- und/oder Axialrichtung der Tropfkammer wirken. Vorzugsweise wirkt die zweite Fügeverbindung wenigstens in Axialrichtung, so dass das Handhabungselement mittels der zweiten Fügeverbindung wenigstens axial an dem Tropfkammeroberteil und/oder dem Tropfkammerunterteil festgelegt ist. Insbesondere für den Fall, dass das Einstechteil unmittelbar an der Tropfkammeranordnung angeordnet und/oder ausgebildet ist, wird durch die formschlüssige zweite Fügeverbindung eine verbesserte Funktionssicherheit bei der Handhabung der Tropfkammeranordnung erreicht. Denn zum Einstechen des Einstechteils in einen Infusionsbehälter oder bei einem Herausziehen des Einstechteils aus demselben sind vergleichsweise hohe axial wirkende manuelle Kräfte erforderlich. Die formschlüssige zweite Fügeverbindung gewährleistet, dass das Handhabungselement hierbei - auch nach einem etwaigen Versagen sonstiger Fügeverbindungen zwischen dem Handhabungselement und den übrigen Komponenten der Tropfkammeranordnung - nicht unbeabsichtigt abgelöst wird.

In weiterer Ausgestaltung der Erfindung ist das Handhabungselement mittels einer stoffschlüssigen dritten Fügeverbindung mit einem Außenwandabschnitt des Tropfkammerunterteils und/oder mit einem Außenwandabschnitt des Tropfkammeroberteils zusammengefügt. Diese Ausgestaltung der Erfindung ermöglicht eine verliersichere Anordnung des Handhabungselements und erlaubt gleichzeitig eine vereinfachte Fertigung, da insbesondere auf eine Anbringung gesonderter Befestigungselemente zur Befestigung des Handhabungselements verzichtet werden kann. Die stoffschlüssige dritte Fügeverbindung kann in einem separaten Fertigungsschritt oder gemeinsam mit der ersten Fügeverbindung ausgebildet sein. Der Außenwandabschnitt des Tropfkammeroberteils ist in Radialrichtung der Tropfkammer außenliegend angeordnet. Entsprechendes gilt für den Außenwandabschnitt des Tropfkammerunterteils.

In weiterer Ausgestaltung der Erfindung ist die dritte Fügeverbindung eine Klebeverbindung oder eine Schweißverbindung. Kleben und/oder Schweißen haben sich als besonders geeignete Verfahren zur Ausbildung der zweiten Fügeverbindung erwiesen.

In weiterer Ausgestaltung der Erfindung ist die dritte Fügeverbindung in einem an die erste Fügeverbindung angrenzenden Bereich angeordnet und bewirkt eine zusätzliche fluiddichte Abdichtung der ersten Fügeverbindung. Bei dieser Ausgestaltung der Erfindung ist das Handhabungselement demnach in einem an die erste Fügeverbindung angrenzenden Bereich angeordnet. Vorzugsweise umgreift das Handhabungselement die erste Fügeverbindung in Umfangsrichtung der Tropfkammer wenigstens abschnittsweise ringförmig. Die erste Fügeverbindung ist in Radialrichtung der Tropfkammer vergleichsweise weiter innenliegend angeordnet als die dritte Fügeverbindung. Diese Ausgestaltung der Erfindung ist besonders vorteilhaft, da eine redundante fluiddichte Abdichtung zwischen dem Tropfkammeroberteil und dem Tropfkammerunterteil erreicht ist.

In weiterer Ausgestaltung der Erfindung weist das Handhabungselement eine in Radialrichtung der Tropfkammer innenliegende Innenkontur auf, die unter Ausbildung eines Umfangsspalts in Radialrichtung von dem Tropfkammeroberteil und/oder dem Tropfkammerunterteil beabstandet ist. Durch den Umfangsspalt kann eine in Radialrichtung begrenzt federelastische Beweglichkeit des Handhabungselements erreicht werden. Hierdurch kann eine gewisse Nachgiebigkeit des Handhabungselements in Radialrichtung erreicht werden, was ergonomisch vorteilhaft ist. Der Umfangsspalt ist in Umfangsrichtung der Tropfkammer erstreckt. Vorzugsweise ist der Umfangsspalt durchgängig über einen gesamten Umfang der Tropfkammer ausgebildet.

Die Erfindung betrifft zudem ein Verfahren zur Herstellung einer Tropfkammeranordnung gemäß der vorstehenden Beschreibung mit: a) Fertigen eines Tropfkammeroberteils; b) Fertigen eines Tropfkammerunterteils; c) fluiddichtes Zusammenfügen des Tropfkammeroberteils und des Tropfkammerunterteils, wobei eine erste Fügeverbindung ausgebildet wird, und wobei eine von dem Tropfkammeroberteil und dem Tropfkammerunterteil umgrenzte Tropfkammer ausgebildet wird.

Die eingangs genannte Aufgabe wird für das Verfahren dadurch gelöst, dass vorgesehen ist: d) Fertigen eines ringförmigen Handhabungselements, wobei das Handhabungselement separat von der ersten Fügeverbindung gefertigt wird, und e) Anordnen des Handhabungselements auf einer Außenseite der Tropfkammer, wobei die Tropfkammer mittels des Handhabungselements in Umfangsrichtung wenigstens abschnittsweise ringförmig umgriffen wird. Durch die erfindungsgemäße Lösung wird gegenüber im Stand der Technik bekannten Verfahren eine vereinfachte und besonders kostengünstige Herstellung ermöglicht, da insbesondere auf ein fertigungstechnisch aufwändiges und investitionsintensives Umspritzen des Tropfkammeroberteils und des Tropfkammerunterteils zur gleichzeitigen Ausbildung der ersten Fügeverbindung und des Handhabungselements verzichtet werden kann. Bei einem solchen Umspritzungsprozess sind die Gestaltungsmöglichkeiten eines zusammen mit der ersten Fügeverbindung auszubildenden Handhabungselements eingeschränkt, da sich der Umspritzungsprozess in erster Linie an den Erfordernissen der fluiddichten ersten Fügeverbindung orientiert. Darüber hinaus müssen hierbei das Tropfkammeroberteil und das Tropfkammerunterteil für die Umspritzung in eine Spritzgießmaschine eingelegt werden. Dies ist aufwändig und führt letztlich zu asynchronen Fertigungsprozessen, die aufwändig aufeinander abgestimmt werden müssen. Demgegenüber ermöglicht die erfindungsgemäße Lösung eine synchrone Fertigung des Tropfkammeroberteils, des Tropfkammerunterteils und des ringförmigen Handhabungselements, wobei die Schritte c) und e) in einem Fertigungs- bzw. Maschinentakt des übrigen Fertigungsprozesses automatisiert ablaufen können. Hinsichtlich der weiteren sich ergebenden Vorteile wird auch auf die diesbezügliche Offenbarung zu der erfindungsgemäßen Tropfkammeranordnung verwiesen, die in entsprechender Weise auch für das erfindungsgemäße Verfahren gilt. Weitere erfindungsgemäße Merkmale und Merkmale erfindungsgemäßer Ausgestaltungen des Verfahrens ergeben sich unmittelbar und eindeutig aus der Beschreibung der erfindungsgemäßen Tropfkammeranordnung und den Ausgestaltungen derselben, so dass zur Vermeidung von Wiederholungen auf die diesbezügliche obenstehende Offenbarung verwiesen wird. So versteht sich, dass das Verfahren insbesondere ein Zusammenfügen des Handhabungselements mit dem Tropfkammeroberteil und/oder dem Tropfkammerunterteil mittels einer stoffschlüssigen dritten Fügeverbindung und/oder einer formschlüssigen zweiten Fügeverbindung umfassen kann.

Weitere Vorteile und Merkmale der Erfindung ergeben sich aus den Ansprüchen sowie aus der nachfolgenden Beschreibung bevorzugter Ausführungsbeispiele, die anhand der Zeichnungen dargestellt sind.
- Fig. 1: zeigt in einer schematischen Längsschnittdarstellung eine Ausführungsform einer erfindungsgemäßen Tropfkammeranordnung mit einem Handhabungselement,
- Fig. 2: in vergrößerter und teilweise abgeschnittener Detaildarstellung die Tropfkammeranordnung nach Fig. 1 in einem Bereich II gemäß Fig. 1 und
- Fig. 3: in einer der Fig. 2 entsprechenden Darstellungsweise eine weitere Ausführungsform einer erfindungsgemäßen Tropfkammeranordnung.

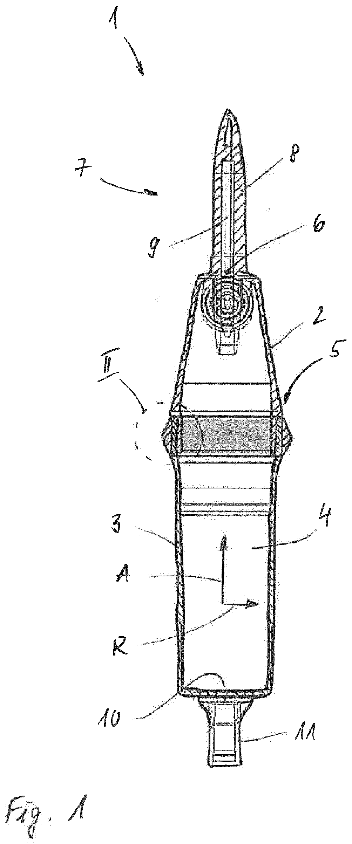

Gemäß Fig. 1 ist eine Tropfkammeranordnung 1 zur Verwendung bei einer Infusionstherapie vorgesehen und insoweit Bestandteil eines im Übrigen nicht näher ersichtlichen medizinischen Infusionssystems.

Die Tropfkammeranordnung 1 weist ein Tropfkammeroberteil 2 und ein Tropfkammerunterteil 3 auf, wobei das Tropfkammeroberteil 2 einer Einlassseite und das Tropfkammerunterteil 3 einer Auslassseite der Tropfkammeranordnung 1 zugeordnet ist. Das Tropfkammeroberteil 2 und das Tropfkammerunterteil 3 sind unter Ausbildung einer Tropfkammer 4 mittels einer fluiddichten ersten Fügeverbindung 5 zusammengefügt. Das Tropfkammeroberteil 2 weist einen Einlass 6 zur Einleitung einer im Rahmen der Infusionstherapie zu verabreichenden medizinischen Flüssigkeit in die Tropfkammer 4 auf. Der Einlass 6 ist fluidleitend mit einem Einstechteil 7 verbunden, das vorliegend einstückig mit dem Tropfkammeroberteil 2 ausgebildet ist.

Bei einer zeichnerisch nicht näher dargestellten Ausführungsform kann das Einstechteil 7 als Komponente des Infusionssystems separat von dem Tropfkammeroberteil 2 angeordnet und auslassseitig mittels einer Schlauchleitung mit dem Einlass 6 fluidleitend verbunden sein.

Das Einstechteil 7 ist auf grundsätzlich bekannte Weise zum Einstechen in einen die zu verabreichende Flüssigkeit beinhaltenden Infusionsbeutel vorgesehen und weist zu diesem Zweck einen hohlen Einstechdorn 8 auf, der mit einem Lumen 9 versehen ist, das in den Einlass 6 mündet. Das Tropfkammerunterteil 3 weist einen über die Tropfkammer 4 fluidleitend mit dem Einlass 6 verbundenen Auslass 10 auf, der vorliegend in einen an dem Tropfkammerunterteil 3 angeordneten Schlauchstutzen 11 mündet, der zur fluidleitenden Verbindung mit einer nicht näher bezeichneten Schlauchleitung des Infusionssystems vorgesehen ist.

Zur Überleitung der zu verabreichenden Flüssigkeit aus dem besagten Infusionsbeutel in die Schlauchleitung wird die Tropfkammeranordnung 1 an einem noch näher zu beschreibenden Abschnitt manuell gegriffen und in Axialrichtung A der Tropfkammer 4 mit dem Einstechdorn 8 voran in den Infusionsbeutel eingestochen. Auf diese Weise kann die zu verabreichende Flüssigkeit aus dem Infusionsbeutel über den Einstechdorn 8 in das Lumen 9 und von dort weiter durch den Einlass 6 in die Tropfkammer 4 und ausgehend von dieser über den Einlass 10 in den Schlauchstutzen 11 und letztlich von dort in die Schlauchleitung gelangen.

Das Tropfkammeroberteil 2 ist vorliegend aus einem formstabilen und blickdurchlässigen Kunststoff gefertigt, was jedoch nicht zwingend ist. Die blickdurchlässige Ausbildung des Tropfkammeroberteils 2 gewährleistet eine einfache visuelle Kontrolle im Hinblick auf eine sich bei der Infusion einstellende Tropfrate. Demgegenüber ist das Tropfkammerunterteil B vorliegend aus einem formnachgiebigen Kunststoff gefertigt, was jedoch ebenfalls nicht zwingend ist. Die formnachgiebige Ausbildung des Tropfkammerunterteils 3 dient einer Pumpfunktion. Hierdurch kann nach Einstechen des Einstechteils 7 in den Infusionsbeutel der vorbeschriebene Durchfluss der zu verabreichenden Flüssigkeit durch entsprechende manuelle Pumpbewegungen an dem Tropfkammerunterteil 3 in Gang gesetzt werden.

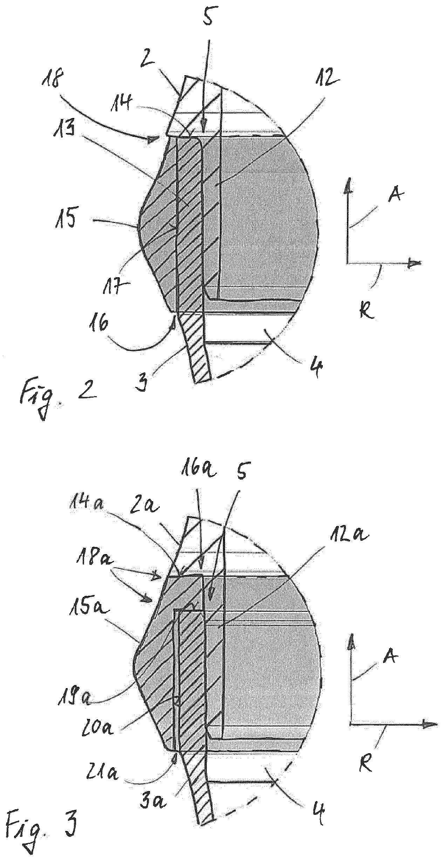

Weitere Einzelheiten der ersten Fügeverbindung 5 zwischen dem Tropfkammeroberteil 2 und dem Tropfkammerunterteil 3 sind anhand Fig. 2 ersichtlich. Das Tropfkammeroberteil 2 weist vorliegend einen in Radialrichtung R der Tropfkammer 4 nach innen abgesetzten und in Axialrichtung A erstreckten Zylinderabschnitt 12 auf. Der Zylinderabschnitt 12 ist zur Ausbildung der ersten Fügeverbindung 5 axial in ein dem Auslass 10 gegenüberliegendes Stirnende des Tropfkammerunterteils 3 eingesteckt. Dementsprechend ist der Zylinderabschnitt 12 in einer nicht näher bezeichneten Umfangsrichtung der Tropfkammer 4 von einem Stirnendabschnitt 13 des Tropfkammerunterteils 3 radial und axial übergriffen. Dabei ist der Stirnendabschnitt 13 vorliegend in Axialrichtung A an einem Radialbund 14 des Tropfkammeroberteils 2 abgestützt. Zum Ausbilden der fluiddichten ersten Fügeverbindung 5 sind der Zylinderabschnitt 12 und der Stirnendabschnitt 13 vorliegend miteinander verklebt, so dass die erste Fügeverbindung in Form einer Klebeverbindung 5 vorliegt. Bei einer nicht dargestellten Ausführungsform kann anstelle einer Klebeverbindung auch eine Schweißverbindung zwischen dem Tropfkammeroberteil 2 und dem Tropfkammerunterteil 3 vorgesehen sein.

Es versteht sich, dass die vorliegende anhand Fig. 2 ersichtliche Detailgestaltung des Tropfkammeroberteils 2 und des Tropfkammerunterteils 3 rein exemplarisch ist. Insbesondere der Zylinderabschnitt 12 ist nicht zwingend. Beispielsweise kann anstelle der vorliegenden Gestaltung vorgesehen sein, dass das Tropfkammeroberteil 2 und das Tropfkammerunterteil 3 zur Ausbildung der ersten Fügeverbindung 5 in Axialrichtung A stumpf zusammengefügt sind. Das heißt ein Einstecken des Tropfkammeroberteils 2 in das Tropfkammerunterteil 3 oder umgekehrt ist zum Ausbilden der fluiddichten ersten Fügeverbindung 5 nicht notwendigerweise erforderlich.

Zur sicheren und ergonomischen Handhabung sowie zur vereinfachten Herstellbarkeit weist die Tropfkammeranordnung 1 erfindungsgemäß ein separat von der ersten Fügeverbindung 5 hergestelltes Handhabungselement 15 auf, das die Tropfkammer 4 in Umfangsrichtung wenigstens abschnittsweise ringförmig umgreift. Vorliegend umgreift das Handhabungselement 15 die Tropfkammer 4 in Umfangsrichtung durchgängig und vollständig ringförmig, was anhand der in Fig. 1 ersichtlichen verdeckten Linien erkennbar ist. Das Handhabungselement 15 ist vorliegend aus Kunststoff gefertigt. Eine besonders ergonomische Handhabung kann erreicht werden, wenn das Handhabungselement 15 in Form einer Weichkomponente aus einem weichelastischen Kunststoff gefertigt ist. Anhand Fig. 1 ist ersichtlich, dass das Handhabungselement 15 eine Außenkontur der Tropfkammeranordnung 1 im Übrigen in Radialrichtung R nach außen überragt. Anders ausgedrückt bildet das Handhabungselement 15 in Radialrichtung R die dickste Stelle der Tropfkammeranordnung 1 und ist auf diese Weise manuell leicht und sicher greifbar.

Das Handhabungselement 15 ist vorliegend axial auf den Stirnendabschnitt 13 des Tropfkammerunterteils 3 aufgeschoben bzw. der Stirnendabschnitt 13 ist in Axialrichtung A in das Handhabungselement 15 eingesteckt. Dabei ist das Handhabungselement 15 mittels einer stoffschlüssigen dritten Fügeverbindung 16 mit einem Außenwandabschnitt 17 des Tropfkammerunterteils 3 zusammengefügt. Der Außenwandabschnitt 17 ist vorliegend an dem Stirnendabschnitt 13 angeordnet. Eine solche Anordnung des Handhabungselements 15 ist nicht zwingend. Bei einer nicht dargestellten Ausführungsform kann das Handhabungselement 15 in Axialrichtung A versetzt zu der ersten Fügeverbindung 5 entweder am Tropfkammeroberteil 2 angeordnet und mit diesem zusammengefügt sein oder stattdessen axial nach unten versetzt abseits des Stirnendabschnitts 13 an dem Tropfkammerunterteil 3 angeordnet und mit diesem zusammengefügt sein.

Die dritte Fügeverbindung ist vorliegend eine Klebeverbindung 16. Anstelle einer Klebeverbindung kann auch eine Schweißverbindung vorgesehen sein.

Die dritte Fügeverbindung 16 erstreckt sich vorliegend in Axialrichtung A und in Umfangsrichtung über eine gesamte Höhe bzw. einen gesamten Innenumfang des Handhabungselements 15 und ist insoweit eine durchgängige Fügeverbindung.

Anhand Fig. 2 ist zudem ersichtlich, dass die dritte Fügeverbindung 16 vorliegend in einem an die erste Fügeverbindung 5 angrenzenden Bereich angeordnet ist, so dass eine zusätzliche fluiddichte Abdichtung der ersten Fügeverbindung 5 bzw. der Tropfkammer 4 mittels der zweiten Fügeverbindung 16 bewirkt ist.

Zudem ist vorliegend eine formschlüssige zweite Fügeverbindung 18 vorgesehen, mittels derer das Handhabungselement 15 mit dem Tropfkammeroberteil 2 zusammengefügt ist. Die zweite Fügeverbindung 18 wirkt vorliegend in Axialrichtung A und ist zwischen einem nicht näher bezeichneten Stirnendabschnitt des Handhabungselements 15 und dem Radialbund 14 des Tropfkammeroberteils 2 ausgebildet. Hierdurch ist das Handhabungselement 15 in Axialrichtung A zusätzlich zu der dritten Fügeverbindung 16 mittels einer formschlüssigen Abstützung an dem Radialbund 14 gehalten.

Anhand Fig. 3 ist eine weitere Ausführungsform einer erfindungsgemäßen Tropfkammeranordnung in dem Bereich II ersichtlich. Die Ausführungsform nach Fig. 3 entspricht im Wesentlichen der zuvor anhand der Fig. 1 und 2 beschriebenen Ausführungsform. Zur Vermeidung von Wiederholungen wird daher auf die Offenbarung zu der Ausführungsform nach den Fig. 1 und 2 verwiesen, die in entsprechender Weise auch für die Ausführungsform nach Fig. 3 gilt. Nachfolgend wird lediglich auf die wesentlichen Unterschiede eingegangen. Dabei sind funktionsgleiche Bauteile und/oder Abschnitte mit gleichen Bezugszeichenziffern versehen und werden bei der Ausführungsform nach Fig. 3 nicht gesondert erläutert. Bauteile und Abschnitte, die jedoch in ihrer konstruktiven Ausführung unterschiedlich sind, sind mit gleichen Bezugszeichenziffern unter Hinzufügung des Kleinbuchstabens a bezeichnet.

Die Ausführungsform nach Fig. 3 sieht insbesondere eine unterschiedlich ausgestaltete dritte Fügeverbindung 16a vor. Die dritte Fügeverbindung 16a ist zwischen einem nicht näher bezeichneten Innenwandabschnitt eines Halteelements 15a und einem nicht näher bezeichneten Außenwandabschnitt eines Zylinderabschnitts 12a eines Tropfkammeroberteils 2a ausgebildet. Die dritte Fügeverbindung ist eine Klebeverbindung 16a.

Zudem ist eine unterschiedlich ausgestaltete formschlüssige zweite Fügeverbindung 18a vorgesehen. Vorliegend ist das Halteelement 15a in Axialrichtung A formschlüssig zwischen einem Radialbund 14a des Tropfkammeroberteils 2a und einer Stirnendfläche 19a des Tropfkammerunterteils 3a festgelegt. Zu diesem Zweck weist das Halteelement 15a einen nicht näher bezeichneten Radialbund auf, der in Radialrichtung R nach innen weisend aufragt.

Weiterhin ist eine in Radialrichtung R innenliegende Innenkontur 20a des Handhabungselements 15a unter Ausbildung eines Umfangsspalts 21a von dem Tropfkammerunterteil 3a beabstandet. Der Umfangsspalt 21a gewährleistet eine begrenzte federelastische Nachgiebigkeit des Handhabungselements 15a in Radialrichtung R.

## Patentansprüche

1. Tropfkammeranordnung (1) für ein medizinisches Infusionssystem, aufweisend ein Tropfkammeroberteil (2, 2a), das einen Einlass (6) zur Einleitung eines medizinischen Fluids in eine Tropfkammer (4) aufweist, und ein Tropfkammerunterteil (3, 3a), das einen Auslass (10) zur Ableitung des medizinischen Fluids aus der Tropfkammer (4) aufweist, wobei das Tropfkammeroberteil (2, 2a) und das Tropfkammerunterteil (3, 3a) mittels einer fluiddichten ersten Fügeverbindung (5) unter Ausbildung der Tropfkammer (4) zusammengefügt sind, wobei ein separat von der ersten Fügeverbindung (5) hergestelltes Handhabungselement (15, 15a) vorgesehen ist, das Handhabungselement (15, 15a) eine gesonderte Komponente der Tropfkammeranordnung (1) ist und mit den übrigen Komponenten der Tropfkammeranordnung (1) zusammengefügt ist, wobei das Handhabungselement (15, 15a) die Tropfkammer (4) in Umfangsrichtung wenigstens abschnittsweise ringförmig umgreift, wobei das Handhabungselement (15, 15a) zum manuellen Greifen eingerichtet ist, und wobei eine Außenkontur des Handhabungselements (15, 15a) die übrigen Komponenten der Tropfkammeranordnung (1) in Radialrichtung der Tropfkammer (4) nach außen überragt, wodurch das Handhabungselement (15, 15a) die dickste Stelle der Tropfkammeranordnung (1) bildet, **dadurch gekennzeichnet dass** die fluiddichte erste Fügeverbindung (5) eine stoffschlüssige Fügeverbindung ist.

2. Tropfkammeranordnung (1) nach Anspruch 1, **dadurch gekennzeichnet, dass** das Handhabungselement (15, 15a) mittels einer formschlüssigen zweiten Fügeverbindung (18, 18a) mit einem Außenwandabschnitt (14, 14a) des Tropfkammeroberteils (2, 2a) und/oder mit einem Außenwandabschnitt (19a) des Tropfkammerunterteils (3a) zusammengefügt ist.

3. Tropfkammeranordnung (1) nach Anspruch 1 oder 2, **dadurch gekennzeichnet, dass** das Handhabungselement (15, 15a) mittels einer stoffschlüssigen dritten Fügeverbindung (16, 16a) mit einem Außenwandabschnitt (17) des Tropfkammerunterteils (3) und/oder mit einem Außenwandabschnitt des Tropfkammeroberteils (2a) zusammengefügt ist.

4. Tropfkammeranordnung (1) nach Anspruch 3, **dadurch gekennzeichnet, dass** die dritte Fügeverbindung (16, 16a) eine Klebeverbindung oder einer Schweißverbindung ist.

5. Tropfkammeranordnung (1) nach Anspruch 3 oder 4, **dadurch gekennzeichnet, dass** die dritte Fügeverbindung (16, 16a) in einem an die erste Fügeverbindung (5) angrenzenden Bereich angeordnet ist und eine zusätzliche fluiddichte Abdichtung der ersten Fügeverbindung (5) bewirkt.

6. Tropfkammeranordnung (1) nach einem der vorhergehenden Ansprüche, **dadurch gekennzeichnet, dass** das Handhabungselement (15a) eine in Radialrichtung (R) der Tropfkammer (4) innenliegende Innenkontur (20a) aufweist, die unter Ausbildung eines Umfangsspalts (21a) in Radialrichtung (R) von dem Tropfkammeroberteil (2a) und/oder dem Tropfkammerunterteil (3a) beabstandet ist.

7. Verfahren zur Herstellung einer Tropfkammeranordnung (1) nach einem der vorhergehenden Ansprüche mit:
a) Fertigen eines Tropfkammeroberteils (2, 2a);
b) Fertigen eines Tropfkammerunterteils (3, 3a);
c) fluiddichtes stoffschlüssiges Zusammenfügen des Tropfkammeroberteils (2, 2a) und des Tropfkammerunterteils (3, 3a), wobei eine erste Fügeverbindung (5) ausgebildet wird, und wobei eine von dem Tropfkammeroberteil (2, 2a) und dem Tropfkammerunterteil (3, 3a) umgrenzte Tropfkammer (4) ausgebildet wird,
d) Fertigen eines ringförmigen Handhabungselements (15, 15a), wobei das Handhabungselement (15, 15a) separat von der ersten Fügeverbindung (5) gefertigt wird, und
e) Anordnen des Handhabungselements (15, 15a) auf einer Außenseite der Tropfkammer (4), wobei die Tropfkammer (4) mittels des Handhabungselements (15, 15a) in Umfangsrichtung wenigstens abschnittsweise ringförmig umgriffen wird, wobei das Handhabungselement (15, 15a) zum manuellen Greifen eingerichtet ist, und wobei eine Außenkontur des Handhabungselements (15, 15a) die übrigen Komponenten der Tropfkammeranordnung (1) in Radialrichtung der Tropfkammer (4) nach außen überragt, wodurch das Handhabungselement (15, 15a) die dickste Stelle der Tropfkammeranordnung (1) bildet.

## Claims

1. Drip-chamber arrangement (1) for a medical infusion system, having a drip-chamber top part (2, 2a), which has an inlet (6) for introducing a medical fluid into a drip chamber (4), and a drip-chamber bottom part (3, 3a), which has an outlet (10) for discharging the medical fluid from the drip chamber (4), wherein the drip-chamber top part (2, 2a) and the drip-chamber bottom part (3, 3a) are joined together by means of a fluid-tight first joining connection (5) to form the drip chamber (4), wherein a handling element (15, 15a) produced separately from the first joining connection (5) is provided, the handling element (15, 15a) being a separate component of the drip-chamber arrangement (1) and joined together with the remaining components of the drip-chamber arrangement (1), wherein the handling element (15, 15a) at least sectionally annularly engages around the drip chamber (4) in a circumferential direction, wherein the handling element (15, 15a) is configured to be gripped manually, and wherein an outer contour of the handling element (15, 15a) projects outwardly beyond the remaining components of the drip-chamber arrangement (1) in a radial direction of the drip chamber (4), whereby the handling element (15, 15a) forms the thickest point of the drip-chamber arrangement (1), **characterized in that** the fluid-tight first joining connection (5) is a materially bonded joining connection.

2. Drip-chamber arrangement (1) according to Claim 1, **characterized in that** the handling element (15, 15a) is joined together with an outer-wall portion (14, 14a) of the drip-chamber top part (2, 2a), and/or with an outer-wall portion (19a) of the drip-chamber bottom part (3a), by means of a form-fitting second joining connection (18, 18a).

3. Drip-chamber arrangement (1) according to Claim 1 or 2, **characterized in that** the handling element (15, 15a) is joined together with an outer-wall portion (17) of the drip-chamber bottom part (3), and/or with an outer-wall portion of the drip-chamber top part (2a), by means of a materially bonded third joining connection (16, 16a).

4. Drip-chamber arrangement (1) according to Claim 3, **characterized in that** the third joining connection (16, 16a) is an adhesive connection or a welded connection.

5. Drip-chamber arrangement (1) according to Claim 3 or 4, **characterized in that** the third joining connection (16, 16a) is arranged in a region adjacent to the first joining connection (5) and gives rise to additional fluid-tight sealing of the first joining connection (5).

6. Drip-chamber arrangement (1) according to one of the preceding claims, **characterized in that** the handling element (15a) has an inner contour (20a) which is situated internally in the radial direction (R) of the drip chamber (4) and which is spaced apart from the drip-chamber top part (2a) and/or the drip-chamber bottom part (3a) in the radial direction (R) so as to form a circumferential gap (21a).

7. Method for producing a drip-chamber arrangement (1) according to one of the preceding claims, comprising:
a) manufacturing a drip-chamber top part (2, 2a);
b) manufacturing a drip-chamber bottom part (3, 3a);
c) joining together the drip-chamber top part (2, 2a) and the drip-chamber bottom part (3, 3a) in a fluid-tight and materially bonded manner, wherein a first joining connection (5) is formed, and wherein a drip chamber (4) delimited by the drip-chamber top part (2, 2a) and the drip-chamber bottom part (3, 3a) is formed,
d) manufacturing a ring-shaped handling element (15, 15a), wherein the handling element (15, 15a) is manufactured separately from the first joining connection (5), and
e) arranging the handling element (15, 15a) on an outer side of the drip chamber (4), wherein the drip chamber (4) is engaged around annularly at least sectionally by means of the handling element (15, 15a) in the circumferential direction, wherein the handling element (15, 15a) is configured to be gripped manually, and wherein an outer contour of the handling element (15, 15a) projects outwardly beyond the remaining components of the drip-chamber arrangement (1) in the radial direction of the drip chamber (4), whereby the handling element (15, 15a) forms the thickest point of the drip-chamber arrangement (1).

## Revendications

1. Agencement de chambre compte-gouttes (1) pour un système de perfusion médicale, présentant une partie supérieure de chambre compte-gouttes (2, 2a) qui présente une entrée (6) pour introduire un fluide médical dans une chambre compte-gouttes (4), et une partie inférieure de chambre compte-gouttes (3, 3a) qui présente une sortie (10) pour évacuer le fluide médical de la chambre compte-gouttes (4), la partie supérieure de chambre compte-gouttes (2, 2a) et la partie inférieure de chambre compte-gouttes (3, 3a) étant assemblées au moyen d'une première liaison d'assemblage (5) étanche aux fluides en réalisant la chambre compte-gouttes (4), un élément de manipulation (15, 15a) fabriqué séparément de la première liaison d'assemblage (5) étant prévu, l'élément de manipulation (15, 15a) étant un composant séparé de l'agencement de chambre compte-gouttes (1) et étant assemblé avec les autres composants de l'agencement de chambre compte-gouttes (1), l'élément de manipulation (15, 15a) entourant la chambre compte-gouttes (4) en direction périphérique au moins par sections en forme d'anneau, l'élément de manipulation (15, 15a) étant adapté pour la préhension manuelle, et un contour extérieur de l'élément de manipulation (15, 15a) dépassant vers l'extérieur les autres composants de l'agencement de chambre compte-gouttes (1) dans la direction radiale de la chambre compte-gouttes (4), l'élément de manipulation (15, 15a) formant ainsi l'emplacement le plus épais de l'agencement de chambre compte-gouttes (1), **caractérisé en ce que** la première liaison d'assemblage (5) étanche aux fluides est une liaison d'assemblage par liaison de matière.

2. Agencement de chambre compte-gouttes (1) selon la revendication 1, **caractérisé en ce que** l'élément de manipulation (15, 15a) est assemblé avec une section de paroi extérieure (14, 14a) de la partie supérieure de chambre compte-gouttes (2, 2a) et/ou avec une section de paroi extérieure (19a) de la partie inférieure de chambre compte-gouttes (3a) au moyen d'une deuxième liaison d'assemblage par liaison de matière (18, 18a).

3. Agencement de chambre compte-gouttes (1) selon la revendication 1 ou 2, **caractérisé en ce que** l'élément de manipulation (15, 15a) est assemblé avec une section de paroi extérieure (17) de la partie inférieure de chambre compte-gouttes (3) et/ou avec une section de paroi extérieure de partie supérieure de chambre compte-gouttes (2a) au moyen d'une troisième liaison d'assemblage à engagement positif (16, 16a).

4. Agencement de chambre compte-gouttes (1) selon la revendication 3, **caractérisé en ce que** la troisième liaison d'assemblage (16, 16a) est une liaison par adhésion ou une liaison par soudage.

5. Agencement de chambre compte-gouttes (1) selon la revendication 3 ou 4, **caractérisé en ce que** la troisième liaison d'assemblage (16, 16a) est agencée dans une zone adjacente à la première liaison d'assemblage (5) et assure une étanchéité supplémentaire aux fluides de la première liaison d'assemblage (5).

6. Agencement de chambre compte-gouttes (1) selon l'une quelconque des revendications précédentes, **caractérisé en ce que** l'élément de manipulation (15a) présente un contour intérieur (20a) situé à l'intérieur dans la direction radiale (R) de la chambre compte-gouttes (4), qui est espacé de la partie supérieure de chambre compte-gouttes (2a) et/ou de la partie inférieure de chambre compte-gouttes (3a) en réalisant un interstice périphérique (21a) dans la direction radiale (R).

7. Procédé de fabrication d'un agencement de chambre compte-gouttes (1) selon l'une quelconque des revendications précédentes, avec :
a) la fabrication d'une partie supérieure de chambre compte-gouttes (2, 2a) ;
b) la fabrication d'une partie inférieure de chambre compte-gouttes (3, 3a) ;
c) l'assemblage étanche aux fluides par liaison de matière de la partie supérieure de chambre compte-gouttes (2, 2a) et de la partie inférieure de chambre compte-gouttes (3, 3a), une première liaison d'assemblage (5) étant réalisée, et une chambre compte-gouttes (4) délimitée par la partie supérieure de chambre compte-gouttes (2, 2a) et la partie inférieure de chambre compte-gouttes (3, 3a) étant réalisée,
d) la fabrication d'un élément de manipulation annulaire (15, 15a), l'élément de manipulation (15, 15a) étant fabriqué séparément de la première liaison d'assemblage (5), et
e) l'agencement de l'élément de manipulation (15, 15a) sur un côté extérieur de la chambre compte-gouttes (4), la chambre compte-gouttes (4) étant entourée en forme d'anneau au moins par sections dans la direction périphérique au moyen de l'élément de manipulation (15, 15a), l'élément de manipulation (15, 15a) étant adapté pour la préhension manuelle, et un contour extérieur de l'élément de manipulation (15, 15a) dépassant vers l'extérieur les autres composants de l'agencement de chambre compte-gouttes (1) dans la direction radiale de la chambre compte-gouttes (4), l'élément de manipulation (15, 15a) formant ainsi l'emplacement le plus épais de l'agencement de chambre compte-gouttes (1).
